# EUROPEAN PATENT APPLICATION

(11) **EP 4 541 717 A2**
(43) Date of publication of application: **23.04.2025**
(21) Application number: 25163025.7
(22) Date of filing: 27.07.2021
(51) Int. Cl.: B64F 5/00

(54) **DUCT ASSEMBLIES FOR AIR MANAGEMENT SYSTEMS AND METHODS OF MANUFACTURE**

(30) Priority: 27.07.2020 US 202063057175 P; 19.05.2021 US 202117324591
(62) Divisional of application: 21188029.9
(71) Applicant: Goodrich Corporation, Charlotte, NC 28217-4578 (US)
(72) Inventor: MOREAU, Claude J., Vernon, 06066 (US); PUJAR, Vijay V., Rancho Santa Fe, CA 92067-5010 (US); POTEET, Steven, Ashland, 01721 (US); SMITH, Blair, South Windsor, 06074 (US); BARTOSZ, Lance R., Granby, 01033 (US); FOLSOM, Michael E., Ellingtom, 06029 (US)
(74) Representative: Dehns

(57) **Abstract**

A method of manufacturing a composite duct for an air management system is described, the method comprising: forming (702) a pre-impregnated material comprising a resin and at least one of a carbon fiber and a glass fiber; laying up (704) the pre-impregnated material into a mold assembly; disposing (706) a surfacing film on an interior surface of the pre-impregnated material; assembling (708) the mold assembly; vacuum bagging (710) the mold assembly; and vacuuming (714) and autoclaving the mold assembly.

## Description

### CROSS-REFERENCE TO RELATED APPLICATIONS

This application is a non-provisional of, and claims priority to, and the benefit of U.S. Provisional Application No. 63/057,175, entitled "DUCT ASSEMBLIES FOR AIR MANAGEMENT SYSTEMS AND METHODS OF MANUFACTURE," filed on July 27, 2020.

### FIELD

The present disclosure relates to air management systems. More specifically, the present disclosure relates to protection of ducts in air management systems.

### BACKGROUND

Pressurized aircraft have integrated air management systems to provide a pressurized environment, fresh air transfer, recycling, heating, and air conditioning to maintain a comfortable, safe environment for occupants for extended periods of time. Air recycling and replacing stale air requires continuous scrubbing for cleanliness to reduce airborne dust, dirt, odors, viruses, spores, and bacteria. This cleaning or scrubbing of the air is typically performed via physical, electrostatic or chemical filtration, such as via a high efficiency particulate air (HEPA) filter. However, bacteria and dirt can accumulate on the filter, benefitting from cleaning or replacement of the filters themselves.

### SUMMARY

An ultraviolet light surface protection system for a duct is disclosed herein. The ultraviolet light surface protection system comprising: an interior surface of the duct; a light source operable to emit a germicidal ultraviolet light into a flow path of the duct defined by the interior surface of the duct to sterilize an air to be provided to a conditioned area; and a coating system disposed on the interior surface, the coating system configured to be ultraviolet resistive, reflective, and anti-microbial.

In various embodiments, the coating system comprises an ultraviolet resistance layer, a reflectivity layer, an anti-microbial layer, and a hydrophobicity layer. The coating system may comprise a quaternary ammonium compound configured to be hydrophobic and anti-microbial. The coating system may be further configured to be hydrophobic. The coating system may comprise a photoactivated metal oxide. The photoactivated metal oxide may comprise at least one of titanium dioxide, zinc oxide or titanium dioxide doped with nitrogen, sulfur or iron. The germicidal ultraviolet light may have a wavelength between about 180 nm and about 280 nm. The coating may comprise a photocatalytic antimicrobial coating.

An air management system of a vehicle having a conditioned area is disclosed herein. The air management system may comprise: a duct having an interior surface defining a flow path for delivering air to the conditioned area; a sterilization system associated with the duct, the sterilization system including a light source operable to emit a germicidal ultraviolet light into the flow path defined by the duct to sterilize the air to be provided to the conditioned area; and a surface protection system for the interior surface of the duct, the surface protection system configured to be ultraviolet resistive, reflective, and anti-microbial.

In various embodiments, the surface protection system may include a coating disposed on the interior surface. The coating may comprise an ultraviolet resistance layer, a reflectivity layer, an anti-microbial layer, and a hydrophobicity layer. The coating may comprise a quaternary ammonium compound configured to be hydrophobic and anti-microbial. The coating may comprise a titanium dioxide based coating. In various embodiments, the coating may be in the form of a surface film. In various embodiments, the coating may comprise more than one surface film, with each surface film comprising of at least one of an ultraviolet resistance layer, a reflectivity layer, an anti-microbial layer, and a hydrophobicity layer. The germicidal ultraviolet light may have a wavelength between about 180 nm and about 280 nm. The air within the duct may have a first flow rate at a first portion of the duct and a second flow rate at a second portion of the duct, the second flow rate being slower than the first flow rate, the light source being positioned to emit the germicidal ultraviolet light within the second portion of the duct. The surface protection system may be integral to the duct.

A method of manufacturing a composite duct for an air management system is disclosed herein. The method may comprise: forming a pre-impregnated material comprising a resin and at least one of a carbon fiber and a glass fiber; laying up the pre-impregnated material into a female mold assembly; disposing one or more surfacing film(s) on an interior surface of the pre-impregnated material; assembling the female mold assembly; vacuum bagging the female mold assembly with a form fitting vacuum bag; and vacuuming and autoclaving the female mold assembly.

In various embodiments, the form fitting vacuum bag may include a complimentary shape to the interior surface to provide a smooth surface finish. In various embodiments, the surfacing film may include a photoactivated catalyst, a metal reflective to ultraviolet light, a quaternary ammonium compound or a resin resistive to degradation by ultraviolet light. The surfacing film may be configured to be ultraviolet resistive, reflective, and anti-microbial. The method may further comprise heating the duct post-cure in a freestanding position.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

### BRIEF DESCRIPTION OF THE DRAWINGS

The subject matter of the present disclosure is particularly pointed out and distinctly claimed in the concluding portion of the specification. A more complete understanding of the present disclosures, however, may best be obtained by referring to the detailed description and claims when considered in connection with the drawing figures, wherein like numerals denote like elements.
FIG. 1 is a schematic diagram of an air management system of an aircraft, in accordance with various embodiments;
FIG. 2 is a perspective view of a portion of the cabin air recirculation sub-system within an aircraft air management system, in accordance with various embodiments;
FIG. 3 is a cross-sectional view an air duct including a sterilization system, in accordance with various embodiments;
FIG. 4 is a cross-sectional view of a portion of a duct of an air management system including a sterilization system, in accordance with various embodiments;
FIG. 5 is a cross-sectional view of a portion of a duct of an air management system including a sterilization system, in accordance with various embodiments;
FIG. 6 is a cross-sectional view of a portion of a duct having a surface protection system, in accordance with various embodiments.
FIG. 7 is a method of manufacturing a duct of an air management system including a sterilization system, in accordance with various embodiments.

### DETAILED DESCRIPTION

The detailed description of exemplary embodiments herein makes reference to the accompanying drawings, which show exemplary embodiments by way of illustration and their best mode. While these exemplary embodiments are described in sufficient detail to enable those skilled in the art to practice the disclosures, it should be understood that other embodiments may be realized, and that logical, chemical, and mechanical changes may be made without departing from the spirit and scope of the disclosures. Thus, the detailed description herein is presented for purposes of illustration only and not of limitation. For example, the steps recited in any of the method or process descriptions may be executed in any order and are not necessarily limited to the order presented. Furthermore, any reference to singular includes plural embodiments, and any reference to more than one component or step may include a singular embodiment or step. Also, any reference to attached, fixed, connected or the like may include permanent, removable, temporary, partial, full and/or any other possible attachment option. Additionally, any reference to without contact (or similar phrases) may also include reduced contact or minimal contact.

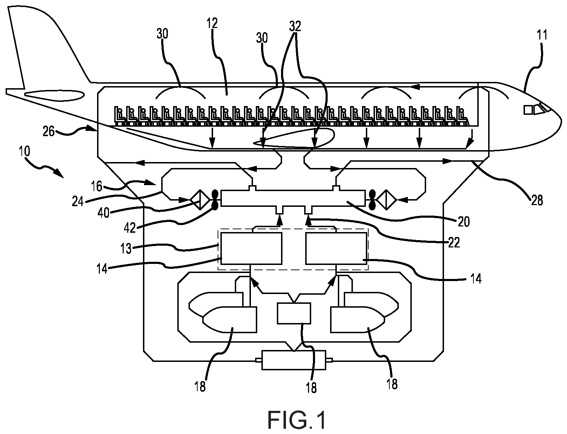

With reference now to FIG. 1, a schematic of an example of an air management system 10 to control the air of a vehicle, such as an aircraft 11 is illustrated. The aircraft 11 includes a pressurized area or cabin 12 that the air management system 10 controls. The cabin 12 may be configured to house people, cargo, and the like therein. The air management system 10 provides conditioned air to, and removes used or contaminated air from, the cabin 12. The air management system 10 includes an environmental control system 13 having at least one air conditioning unit or pack 14, and a cabin air recirculation sub-system 16. While the air management system 10 is illustrated and described herein with reference to an aircraft 11, it should be understood that the systems and techniques discussed herein may be used for a variety of air management systems 10. For example, the cabin 12 may be replaced with any closed volume to be conditioned. As such, systems described herein may be used with ship air management systems, such as submarines and cruise liners for example, personnel carrier air management systems, bus, trolley, train, or subway air management systems, or any other air management system that requires a continual supply of conditioned air.

As shown in the FIG. 1, a medium, such as air for example, is provided from one or more sources 18 to the air management system 10. Examples of suitable sources 18 include but are not limited to an engine of the aircraft 11 and an auxiliary power unit of the aircraft 11. The medium output from these sources 18 is provided to the one or more air conditioning units 14 of the environmental control system 13. Within these air conditioning units 14, the medium is conditioned. This conditioning includes altering one or more of a pressure, temperature, humidity, or flow rate of the medium based on an operating condition of the aircraft. The medium output or discharged from the one or more air conditioning units 14 of the environmental control system 13 may be used maintain a target range of pressures, temperatures, and/or humidity within the cabin 12.

In an embodiment, the mixed medium is delivered to the cabin 12 from the air mixing unit 20 via an air distribution system 26 including one or more conduits 28. As shown, the mixed medium may be delivered to the cabin 12 and cockpit via a ventilation system arranged near a ceiling of the cabin 12. In various embodiments, the mixed medium typically circulates from the top of the cabin 12 toward the floor and is distributed to a plurality of individual vents 30 of the ventilation system spaced laterally between the front and rear of the cabin 12. It should be understood that the air management system 10 illustrated and described herein is intended as an example only, and that any suitable air management system is within the scope of the disclosure.

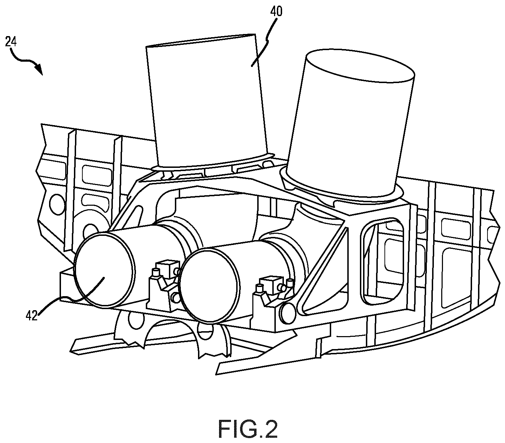

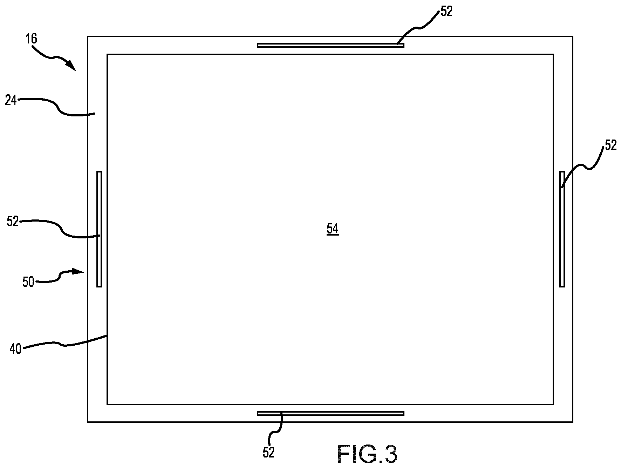

With reference now to FIG. 2, an example of a portion of the cabin air recirculation sub-system within the air management system 10 is shown in more detail. In the illustrated, non-limiting embodiment, a portion of the duct 24 of the cabin air recirculation sub-system 16 fluidly connects one or more inlets 32 (see FIG. 1) of the cabin 12 to the air mixing unit 20. Mounted within the duct is a filter 40 configured to remove bacteria, viruses and particulate matter from the cabin recirculation air provided from the inlets 32 in the cabin 12 as it flows through the filter 40. Although the filter 40 is shown as being arranged adjacent a downstream end of the duct, such as directly upstream from an interface between the duct and the air mixing unit, a filter arranged at any location within the duct is contemplated herein. Further, although the filter 40 is illustrated as having a circular configuration in FIG. 2, and a rectangular configuration in FIG. 3, it should be understood that a filter 40 having any configuration is within the scope of the disclosure. In various embodiments, the filter 40 is a high-efficiency particulate air (HEPA) type filter. However, any suitable filter, or combination of multiple filters is within the scope of the disclosure. Further, in an embodiment, the duct 24 includes a recirculation fan 42 to establish an overpressure that is used to drive the flow of the recirculating cabin air through the filter 40 and to the air mixing unit 20. However, embodiments of a portion of a cabin air recirculation sub-system 16 that do not include a fan such that air flow through the duct 24 is driven by another source or by pressure for example, are also contemplated herein.

With reference now to FIGS. 3-5, in an embodiment, the air management system 10 additionally includes a sterilization system 50 for sterilizing at least a portion of the air therein. In addition to the removal of particulate matter, the sterilization described herein additionally includes killing or rendering harmless bacteria or airborne viruses within the air management system 10 and/or an air flow there through. Because dehumidified air is more readily sterilized, the air is dehumidified before passing through (upstream from) the sterilization system 50 and is then re-humidified downstream of the sterilization system 50, such as in the air mixing unit 20 for example.

As shown, in various embodiments, the sterilization system 50 is used to sterilize a portion of the air provided to the cabin 12, such as the cabin recirculation air discharged from inlets 32 of the cabin 12 and provided to the air mixing unit 20 and/or a portion of one or more ducts 24 extending between the cabin inlets 32 and the air mixing unit 20. Further, it should be understood that although a duct 24 of the cabin air recirculation sub-system 16 is illustrated and described herein with respect to the sterilization system 50, any portion of the air management system 10, and specifically any portion or duct that is used to move cabin discharge air or cabin recirculation air through the air management system 10, including but not limited to the air mixing unit 20 and the conduits 28 of the air distribution system 26 for example, may be adapted for use with a sterilization system 50 as described herein.

The sterilization system 50 includes at least one light source 52 capable of emitting a light having a wavelength suitable to perform germicidal irradiation. In an embodiment, the light source 52 is operable to emit a germicidal ultraviolet light, such as having a wavelength between about 180 and about 280 nanometers, also known as "UV-C." It should be understood that ultraviolet light having another wavelength, such as between 280nm and 400nm, and more specifically between 280nm and 315nm, or other types of light may also be suitable for use in sterilization applications. Additionally, a light source 52 having any configuration, such as an individual bulb, a light strip having a plurality of bulbs or light emitting diodes, or another type of emitter, is within the scope of the disclosure. In embodiments of the sterilization system 50 including a plurality of the light source 52, a configuration of the light sources may be substantially identical or may vary based on a position of the light source 52 relative to the air management system.

The use of germicidal ultraviolet light, and specifically UV-C light, typically employs exposure for only a matter of seconds to kill all or substantially all virus or bacteria present. However, the length of exposure may vary in response to one or more parameters, such as the wavelength of the light, the intensity or strength of the light, the volume flow rate of air, and the humidity of the air, for example. In various embodiments, the one or more light sources 52 and an intensity of each light source 52 is determined based on at least one of the volume flow rates and the humidity of the air. Because exposure for only a limited period of time is needed for sterilization, the one or more light sources 52 may be disposed at one or more areas along the flow path defined by the duct 24.

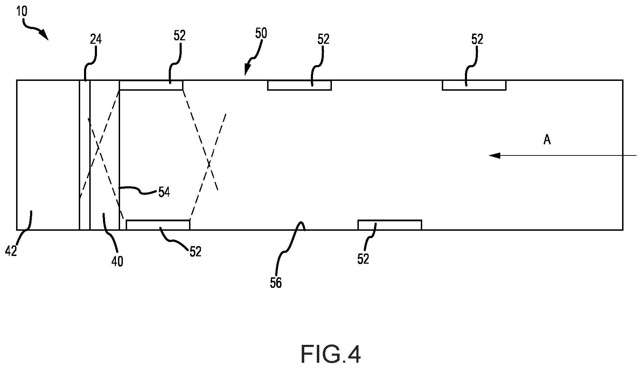

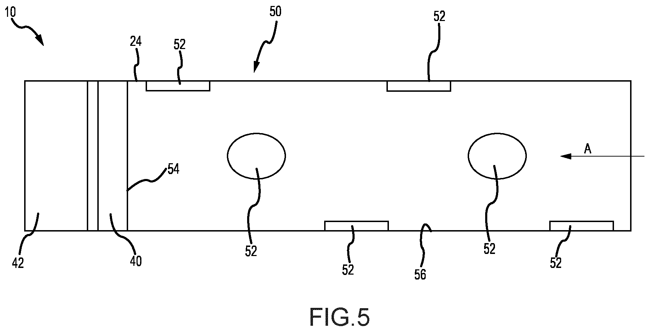

In various embodiments, the one or more light sources 52 are located at an area of the flow path where the flow of air provided from the cabin inlets 32 is slowest. For example, the flow rate of the cabin recirculation air through the portion of the duct 24 including the filter 40 is reduced relative to the flow rate of the air at an upstream portion of the duct 24 to maximize the efficacy of the filter 40. Accordingly, in various embodiments, one or more light sources 52 are mounted such that the light emitted therefrom projects over at least a portion, and in some embodiments, over substantially the entire surface 54 of the filter 40. As a result, any viruses or bacteria present on the filter 40, such as trapped in the filter material itself, are killed or neutralized. In such embodiments, the one or more light sources 52 may be integrated into the filter 40 (FIG. 3) and/or may be mounted to a portion of the duct 24, such as directly adjacent the filter 40 (FIG. 4), or alternatively, at a location axially offset from the filter 40 (FIG. 5) such that the light emitted from the light sources 52 overlaps the surface the filter 40.

In various embodiments, the sterilization system 50 may additionally include one or more light sources 52 arranged at a location where the flow rate of the cabin circulation air flow A is faster than at the filter 40. As shown, one or more light sources 52 may be arranged within the air management system 10 to emit germicidal ultraviolet light over a portion of the flow path defined by the duct 24, upstream from the filter 40, as shown in FIGS. 4 and 5. Although the figures show a sterilization system 50 including a plurality of light sources 52 operable to illuminate substantially the entire length of the duct 24 extending between an upstream end thereof 56 and the filter 40, embodiments where only a portion of the duct 24 is illuminated are also contemplated herein. In various embodiments including multiple light sources 52, each of the plurality of light sources 52 may be positioned such that the light emitted therefrom overlaps with the light emitted from an adjacent light source 52. As shown, the light sources may be mounted within the same plane, such as adjacent the same side of the duct 24, or in various embodiments, at different sides of the duct 24, such as opposite sides (FIG. 4) or adjacent sides (FIG. 5) for example. As a result, the region of the duct 24 illuminated by the light sources 52 will be free from shadows or non-illuminated areas where bacteria or viruses may accumulate.

By mounting one or more light sources 52 capable of emitting a germicidal ultraviolet light along the flow path of the cabin recirculation air, the light sources 52 may be used to continuously disinfect the airflow and/or a portion of a duct 24, without exposing aircraft occupants to any harmful effects from exposure to a high intensity ultra-violet light. Further, the sterilization system could continuously operate when the vehicle is both airborne and grounded without the need for any chemical means of rendering airborne viruses and bacteria harmless. Additionally, the one or more ultra-violet light sources 52 are small, use minimal power, and do not require high power, heat, or chemicals to kill viruses and bacteria.

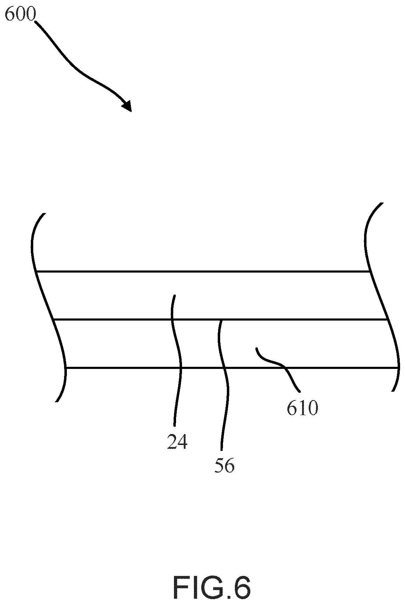

In various embodiments, the duct 24 may comprise a composite material. A "composite material," as described herein may comprise any composite material, such as carbon fiber, fiber-reinforced polymer (e.g., fiber glass), para-aramid fiber, aramid fiber, fiber reinforced epoxy, and/or carbon fiber-reinforced bismaleimide (BMI). In various embodiments, the duct 24 comprises fiber reinforced epoxy or BMI. In various embodiments, utilization of UV light, as disclosed herein, may degrade a composite material, specifically those that contain high degrees of aromaticity. As such, referring now to FIG. 6, a surface protection system 600 for a duct 24 is illustrated, in accordance with various embodiments. In various embodiments, the interior surface 56 of the duct 24 within the region illuminated by the one or more light sources 52 (from FIGs. 3-5) may have a coating system 610.

In various embodiments, the coating system 610 may be configured for UV resistance, reflectivity, anti-microbial, and/or hydrophobicity. For example, with respect to UV resistance in accordance with various embodiments, the coating system 610 may comprise an anti-UV polymer stabilizer, such as benzotriazoles and hydroxyphenyl triazines, oxanilides, and/or benzophenones. With respect to reflectivity, in accordance with various embodiments, the coating system 610 may include a reflected or mirrored coating, such as one or more of aluminum, gold, chrome, nickel, titanium, copper, silver, copper oxide, titanium dioxide, zinc oxide, or another suitable shiny material or polished surface. With respect to anti-microbial in accordance with various embodiments, the coating system 610 may comprise a photocatalytic antimicrobial coating, such as a titanium dioxide based coating, a NiO/SrBi2O4 based coating, a zinc oxide based coating, or the like. With respect to hydrophobicity, the coating system 610 may comprise manganese an oxide polystyrene composite based coating, a zinc oxide polystyrene composite based coating, a precipitated calcium carbonate based coating, a carbon nanotube based coating, a silica sol-gel coating, a fluorinated silane coating, and/or a fluoropolymer coating. In various embodiments, the coating system 610 may be multi-functional. For example, a quaternary ammonium compound, such as hexadecyltrimethylammonium ('cetrimide'), chlorhexidine, and a benzalkonium chloride (number of carbon atoms in the alkyl chain (n) = 8 - 18), may provide both hydrophobicity and anti-microbial functionality.

In various embodiments, the titanium dioxide based coating may be a doped titanium dioxide based coating. The dopants in the titanium dioxide coating may comprise at least one of iron, sulfur, or nitrogen. A reflectivity layer may comprise a metal, wherein the metal may comprise of aluminum to improve the reflectivity to ultraviolet light. In various embodiments, the reflectivity layer may comprise aluminum having one or more dielectric layer films to enhance the reflectivity. In various embodiments, each dielectric film layer may comprise of at least one of a polycarbonate or an oxide. In various embodiments, the oxide layer may comprise of silicon dioxide.

Further, in accordance with various embodiments, coating system 610 may be applied via any suitable method, such as via a spray, dip, wipe, vapor deposition, plating, or other known method. In an embodiment, the coating material is applied via vapor deposition, such as via atomic layer deposition for example. Application of a coating material via atomic layer deposition permits non-line-of-sight coating because a molecular layer of various germicidal chemical compounds may be formed anywhere the vapor makes contact.

In various embodiments, the coating system 610 may be a multi-layered coating system. For example, the coating system 610 may comprise a UV resistance layer, a reflectivity layer, an anti-microbial layer, and a hydrophobicity layer. In various embodiments, a single coating may include all or most of the functionality as outlined above. In various embodiments, the coating system 610, as described herein, may be configured to protect the interior surface 56 of the duct 24 from degradation due to exposure to UV light, from microbes, and/or provide more efficient airflow through the system. In various embodiments, the UV resistance, reflectivity, anti-microbial, and/or hydrophobicity may be achieved through additive in the composite, in accordance with various embodiments.

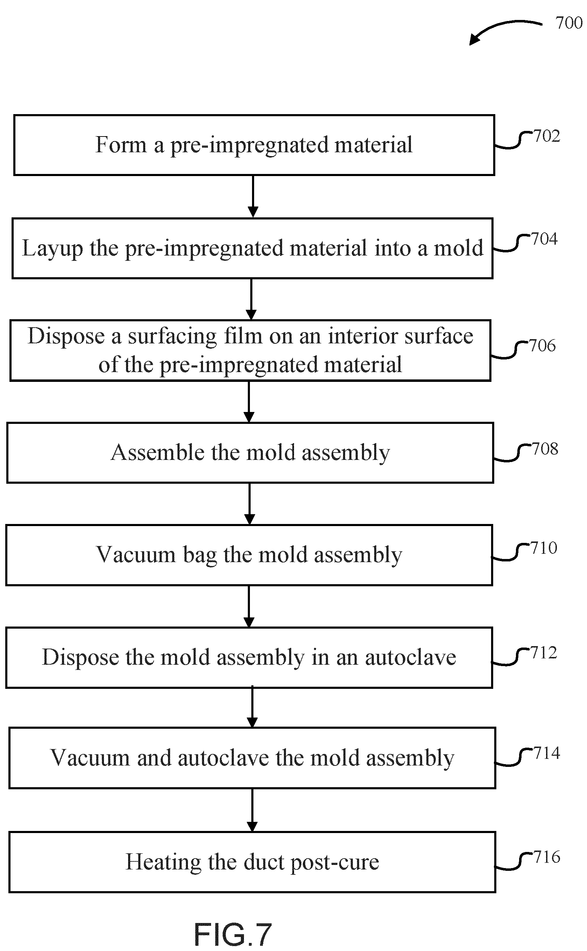

For example, referring now to FIG. 7, a method of manufacturing a duct having a surface protection system is illustrated, in accordance with various embodiments. In various embodiments, the method 700 may comprise forming pre-impregnated material (step 702). The pre-impregnated material may be formed by impregnating a carbon or a glass fiber with a resin. In various embodiments, the resin may include an epoxy or the like. The method 700 may further comprise laying up the pre-impregnated material into a mold (step 704). In various embodiments, the mold may comprise a female mold or a male mold. In various embodiments, the mold may include a complimentary shape to a duct. In various embodiments, the duct may be in accordance with duct 24 from FIGs. 3-6.

In various embodiments, the method 700 may further comprise disposing a surfacing film on an interior surface of the pre-impregnated material (step 706). In various embodiments, the surfacing film may be configured in accordance with the coating system 610 from FIG. 6. For example, the surfacing film may be configured for UV resistance, reflectivity, anti-microbial, and/or hydrophobicity. For example, in accordance with various embodiments, photoactivated metal oxide, such as a titanium dioxide based coating, may be injected into the surfacing film prior to disposing the surfacing film on the interior surface. The method 700 may further comprise assembling the mold (step 708).

In various embodiments, the method 700 may further comprise vacuum bagging the mold assembly (step 710). In various embodiments, vacuum bagging the mold assembly may include utilizing a form fitting vacuum bag. A form fitting vacuum bag, as described herein is a vacuum bag foam, or the like, having a complimentary shape to an interior surface of the duct. In this regard, the vacuum bagging process may provide a smoother surface finish relative to typical vacuum bagging applications. In this regard, a smoother surface may provide a more reflective surface. In various embodiments, a smooth surface as described herein includes a surface roughness between 0 and 8 µin. (0 and 0.2 µm) or between 0 and 4 µin. (0 and 0.1 µm). In various embodiments, reflective, as described herein refers to a glossmeter between 50 GU and 100 GU or between 70 GU and 100 GU. A more reflective surface may more effectively reflect the UV light and/or more effectively disinfect the airflow and/or a portion of a duct 24 while the sterilization system is in use.

In various embodiments, the method 700 further comprises placing the mold assembly under vacuum and an autoclave pressure at a temperature up to 350 °F (177 °C) for about six hours (step 714). In various embodiments, step 714 may result in a cured composite duct with a coating system, as illustrated in FIG. 6. After the autoclave process, the method 700 further comprises removing the cured composite duct from the mold assembly and heating the duct in a freestanding position at a temperature up to 350 °F (177 °C) for about six hours.

In various embodiments, the method 700 may produce a duct assembly having a surface protection system in accordance with FIG. 6. In this regard, the duct assembly may be configured to survive exposure to UV sterilization with little to no degradation. The duct assembly may include anti-microbial and reflective functionality to enhance the disinfecting capability of the UV light and the exposure of the UV light of the air management system. In various embodiments, the duct assembly may be laid up using resin pressure molding in a closed mold.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosures.

The scope of the disclosures is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods and apparatus are provided herein. In the detailed description herein, references to "one embodiment", "an embodiment", "an example embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiment

Furthermore, no element, component, or method step in the present disclosure is intended to be dedicated to the public regardless of whether the element, component, or method step is explicitly recited in the claims. As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

## Claims

1. A method of manufacturing a composite duct for an air management system, the method comprising:
forming (702) a pre-impregnated material comprising a resin and at least one of a carbon fiber and a glass fiber;
laying up (704) the pre-impregnated material into a mold assembly;
disposing (706) a surfacing film on an interior surface of the pre-impregnated material;
assembling (708) the mold assembly;
vacuum bagging (710) the mold assembly; and
vacuuming (714) and autoclaving the mold assembly.

2. The method of claim 1, wherein:
the mold assembly is a female mold assembly,
vacuum bagging (710) the mold assembly includes vacuum bagging the mold assembly with a form fitting vacuum bag, and
the form fitting vacuum bag includes a complimentary shape to the interior surface.

3. The method of claim 1 or 2, wherein the surfacing film includes at least one of a photoactivated catalyst, a metal reflective to ultraviolet light, a quaternary ammonium compound or a resin resistive to degradation by ultraviolet light.

4. The method of any of claims 1 to 3, wherein the surfacing film is configured to be ultraviolet resistive, reflective, and anti-microbial.

5. The method of any of claims 11to 4, further comprising heating the duct post-cure in a freestanding position.
